# FASCICULE DE BREVET EUROPEEN

(11) **EP 1 811 994 B1**
(45) Date de publication et mention de la délivrance du brevet: **25.06.2008**
(21) Numéro de dépôt: 05810755.8
(22) Date de dépôt: 10.11.2005
(51) Int. Cl.: A61K 31/409, A61K 41/00, C07D 487/22, A61P 35/00

(54) **NOUVEAUX DERIVES DE PORPHYRINE, NOTAMMENT CHLORINES ET BACTERIOCHLORINES ET LEURS APPLICATIONS EN THERAPIE PHOTODYNAMIQUE**
NEUE DERIVATE VON PORPHYRIN, INSBESONDERE CHLORINE UND BACTERIOCHLORINE UND IHRE VERWENDUNGEN IN DER PHOTODYNAMISCHEN THERAPIE
NOVEL DERIVATIVES OF PORPHYRIN, PARTICULARLY CHLORINS AND BACTERIOCHLORINS, AND USES THEREOF IN PHOTODYNAMIC THERAPY

(30) Priorité: 16.11.2004 FR 0412149
(43) Date de publication de la demande: 01.08.2007
(73) Titulaire: Universidade de Coimbra, 3004-531 Coimbra (PT)
(72) Inventeur: MIGUENS PEREIRA, Maria, Rua Larga. 3004-535 Coimbra (PT); ARNAUT MOREIRA, Luis, Guilherme, Rua Larga- 3000Coimbra (PT); FORMOSINHO SIMÖES, Sebastiäo, José, P-3004-531 Coimbra (PT); MONTEIRO, Carlos, 3060-115 Cadima (PT)
(74) Mandataire: Duthoit, Michel Georges André
(86) Numéro de dépôt international: PCT/EP2005/012212
(87) Numéro de publication internationale: WO 2006/053707

(56) Documents cités:
- WO-A-98/50386
- WO-A-03/008430
- WO-A-03/020309
- US-A- 5 876 989

## Description

La thérapie photodynamique (PDT) est une technique utilisée pour traiter plusieurs types de maladies, notamment certains types de cancer. Cette technique consiste à marquer les tissus pathologiques avec un agent photosensibilisant, puis à provoquer la destruction sélective de ces tissus en les exposant à une source lumineuse avec une longueur d'onde spécifique. Cette lumière monochromatique est généralement produite par un laser ou une diode laser.

Par agent photosensibilisant, on entend toute molécule susceptible d'emmagasiner de l'énergie lumineuse, d'être activée par elle et de se prêter ainsi à de nombreuses combinaisons biochimiques.

L'agent photosensibilisant, généralement entre 0,1 et 0,5 micromole/kg de masse corporelle, est administré au patient, puis capté par les cellules de l'ensemble de l'organisme. La molécule photosensibilisante s'accumule préférentiellement dans les cellules cancéreuses, mais demeure inactive jusqu'à son exposition à une lumière de longueur d'onde appropriée. L'illumination ou irradiation de la tumeur par la lumière active la molécule, qui interagit alors avec l'oxygène et forme une substance transitoire, notamment l'oxygène singulet. L'oxygène singulet est une molécule très réactive et toxique qui détruit les cellules cancéreuses dans lesquelles l'agent photosensibilisant s'est concentré.

Un certain délai s'écoule entre l'administration de l'agent photosensibilisant et son activation au moyen de la lumière laser. La lumière laser utilisée en thérapie photodynamique est focalisée au moyen d'une fibre optique et n'est appliquée que pendant quelques minutes. Le médecin maintient la fibre optique à proximité immédiate du cancer afin d'administrer la quantité correcte de lumière. De ce fait, la thérapie photodynamique ne lèse les cellules saines que de façon minimale.

Au stade précoce d'un cancer, l'objectif de cette technique peut être d'éliminer totalement et de guérir le cancer mais, au stade avancé, il peut être de réduire le volume de la tumeur afin de soulager des symptômes. De nouvelles cellules normales remplacent celles détruites par la thérapie photodynamique, ce qui permet une cicatrisation rapide après traitement et évite les cicatrices particulièrement disgracieuses qui peuvent se former avec d'autres formes d'ablation des tissus.

Même les patients déjà traités par chirurgie, radiothérapie ou chimiothérapie peuvent être soumis à cette technique de façon sûre.

Comme exemple de maladies pouvant être traitées par la thérapie photodynamique, nous pouvons notamment citer le cancer de l'estomac, de l'intestin, du poumon, du sein, de l'utérus, de l'oesophage, de l'ovaire, du pancréas, du foie, de la vessie, de la bile, de la langue, du cerveau, de la peau, de la thyroïde, de la prostate, de la glande parotide, ainsi que certaines maladies virales et/ou microbiennes.

La thérapie photodynamique peut également être utilisée comme moyen de diagnostic de certaines formes de cancer. A cette fin, il suffit que les molécules photosensibilisantes soient fluorescentes et, donc, capable d'émettre de la lumière lorsqu'elles reçoivent un rayonnement.

Actuellement, il est connu l'utilisation de certains dérivés de porphyrine, notamment l'hématoporphyrine, comme agents photosensibilisants en thérapie photodynamique. Cet agent, connu sous la marque Photofrin, déposée par la société AXCAN PHARMA INC., est un mélange purifié d'hématoporphyrine. L'hématoporphyrine est à son tour un dérivé de l'hémoglobine porcine.

D'ailleurs, le Photofrin® est la seule molécule photosensibilisante jusqu'aujourd'hui, à avoir reçu l'autorisation de commercialisation dans plusieurs pays et, notamment en France, pour le traitement du cancer de l'oesophage.

Malgré les avantages présentés par le Photofrin®, tels que sa solubilité en milieux aqueux, un bon rendement de formation d'oxygène singulet et sa facilité de synthèse, il présente toutefois quelques inconvénients.

Tout d'abord, le Photofrin® est activé par une lumière de 630 nm. Or, à cette longueur d'onde, la pénétration de la lumière dans les tissus n'est que de 5 à 10 mm, ce qui constitue un sérieux handicap lorsque les tumeurs sont plus larges et plus profondes. En outre, cet agent photosensibilisant provoque une photosensibilité cutanée jusqu'à six semaines après le traitement. Enfin, le fait que le Photofrin® soit un mélange de plusieurs molécules, rend plus difficile le choix de la dosimétrie appropriée, tant du photosensibilisant que de la lumière administrée.

WO03/020309 décrit des dérivés de porphyrine et leurs applications en thérapie photodynamique.

Parmi d'autres composés en cours d'étude, appelés composés de seconde génération, le document WO-98/50386 décrit certains dérivés de la benzoporphyrine. Ces dérivés présentent certains avantages par rapport au Photofrin® en ce qu'ils absorbent la lumière à une longueur d'onde de 690 nm et, par conséquent, peuvent être utilisés dans le traitement des cancers plus larges et plus profonds. Ces dérivés présentent en outre une meilleure sélectivité envers les cellules cancéreuses.

Cela étant, certains problèmes liés à la sélectivité, à l'absorption de la lumière et à la toxicité persistent.

Le but de la présente invention est de proposer de nouveaux dérivés de porphyrine, tels que notamment chlorines et/ou bactériochlorines, qui pallient les inconvénients précités, notamment en ce qui concerne l'accumulation préférentielle dans les cellules cancéreuses, une meilleure stabilité et une plus faible phototoxicité.

Un autre but de la présente invention est de proposer un médicament anti-cancéreux et/ou anti-viral et/ou anti-microbien afin d'être utilisé en thérapie photodynamique.

La présente invention concerne un dérivé de porphyrine, notamment chlorine, de formule : 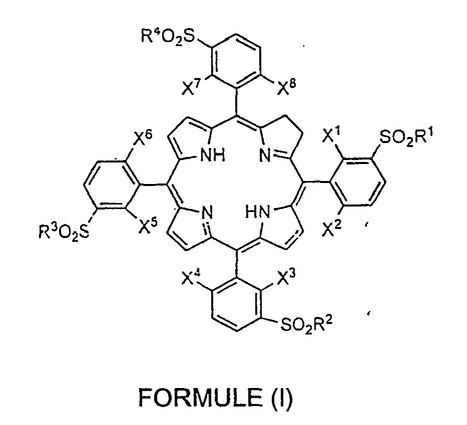 dans laquelle :
- X¹, X², X³, X⁴, X⁵, X⁶, X⁷, X⁸ sont chacun, identiques ou différents, choisis parmi les atomes d'halogène, et/ou les atomes d'hydrogène ;
- R¹, R², R³, R⁴ sont chacun, identiques ou différents, choisis parmi les groupements : -OH, acides aminés, -OR et -NHR et/ou atome de chlore, dans lesquels R est un groupement alkyle ayant de 1 à 12 atomes de carbone.

La présente invention concerne également un dérivé de porphyrine, notamment bactériochlorine, de formule : 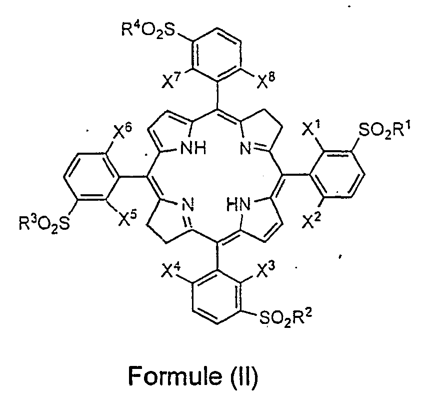 dans laquelle :
- X¹, X², X³, X⁴, X⁵, X⁶, X⁷, X⁸ sont chacun, identiques ou différents, choisis parmi les atomes d'halogène, et/ou les atomes d'hydrogène ;
- R¹, R², R³, R⁴ sont chacun, identiques ou différents, choisis parmi les groupements : -OH, acides aminés, -OR et -NHR et/ou atome de chlore, dans lesquels R est un groupement alkyle ayant de 1 à 12 atomes de carbone.

Un des avantages de ces dérivés réside dans la présence du groupement chlorosulfonyl dans la molécule, telle que décrite dans la présente invention, ce qui permet d'introduire d'une façon simple et efficace une grande variété de groupements chimiques.

La présente invention concerne, en outre, un médicament anti-cancéreux et/ou anti-viral et/ou anti-microbien à usage humain ou animal présentant comme principe actif principal un ou plusieurs composés décrits dans la présente invention.

L'invention sera mieux comprise à la lecture de la description suivante, accompagnée des dessins en annexe parmi lesquels :
- la figure 1 représente le spectre d'absorption de la chlorine selon la formule (I) en solution aqueuse avant (ligne pleine) et après (ligne pointillée) irradiation de l'échantillon par la deuxième harmonique d'un laser Nd:YAG ;
- la figure 2 représente le spectre d'absorption de la bactériochlorine selon la formule (II) en solution aqueuse avant (ligne pleine) et après (ligne pointillée) irradiation de l'échantillon par la deuxième harmonique d'un laser Nd :YAG.

La présente invention concerne tout d'abord un dérivé de porphyrine, notamment chlorine, de formule : 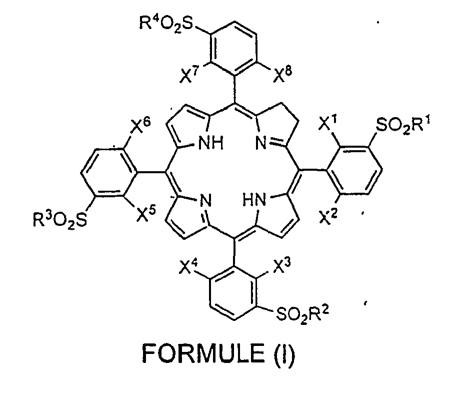 dans laquelle :
- X¹, X², X³, X⁴, X⁵, X⁶, X⁷, X⁸ sont chacun, identiques ou différents, choisis parmi les atomes d'halogène, et/ou les atomes d'hydrogène ;
- R¹, R², R³, R⁴ sont chacun, identiques ou différents, choisis parmi les groupements : -OH, acides aminés, -OR et -NHR et/ou atomes de chlore, dans lesquels R est un groupement alkyle ayant de 1 à 12 atomes de carbone.

Un des avantages de ce type de dérivés réside dans le fait qu'ils sont amphiphiles. Le caractère amphiphile est essentiel pour que les molécules puissent traverser la membrane cellulaire et s'accumuler à l'intérieur des cellules.

Par amphiphile, on entend toute molécule qui présente à la fois un caractère hydrophile et hydrophobe.

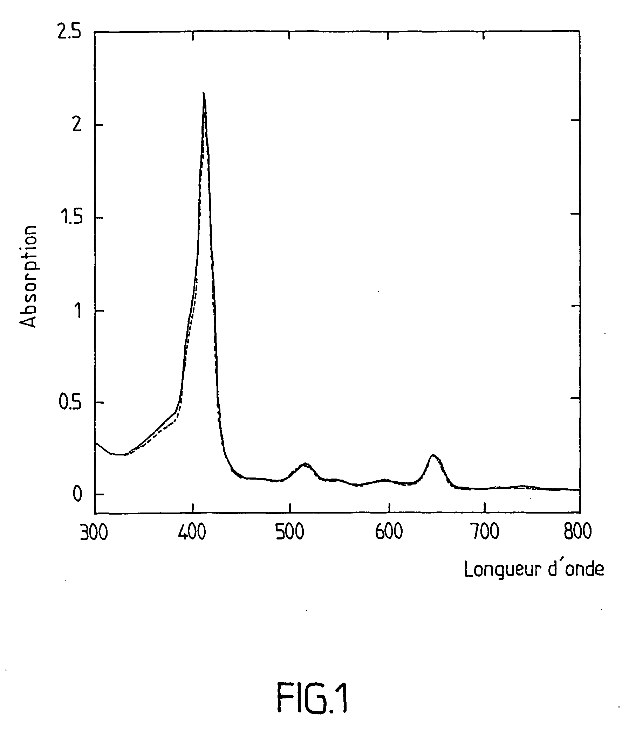

Ce type de dérivés présente une bande d'absorption à 650 nm, telle que montré à la figure 1.

La présente invention concerne également les dérivés répondant à la formule (I), dans laquelle :
- X¹, X², X³, X⁴, X⁵, X⁶, X⁷, X⁸ sont chacun, identiques ou différents, choisis parmi les atomes d'halogène, et/ou les atomes d'hydrogène, tels qu'au moins un des radicaux X de chaque paire X¹/X², X³/ X⁴, X⁵/X⁶, X⁷/X⁸ est un atome d'halogène.

Un avantage de ce type particulier de dérivés réside dans la présence des atomes d'halogène dans les positions ortho du groupement phényl qui augmente le rendement quantique de formation de l'état triplet, sans pour autant diminuer significativement sa durée de vie. Avec un rendement quantique plus élevé, l'état triplet peut transférer son énergie plus efficacement vers l'oxygène ayant comme conséquence un rendement de formation de l'oxygène singulet plus élevé.

En outre, les atomes d'halogène dans les positions ortho du groupement phényl rendent aussi le macrocycle tetrapyrolique plus stable et, donc, plus efficace dans son utilisation en thérapie photodynamique.

Encore selon la présente invention, et conformément à la formule (I) :
- X², X⁴, X⁶ et X⁸ sont soit des atomes de chlore, soit des atomes de fluor ;
- X¹, X³, X⁵ et X⁷ sont des atomes d'hydrogène,
- R¹, R², R³, R⁴ sont des atomes de chlore,

Selon une autre variante de la présente invention, et conformément à la formule (I) :
- X², X⁴, X⁶ et X⁸ sont soit des atomes de chlore, soit des atomes de fluor ;
- X¹, X³, X⁵ et X⁷ sont des atomes d'hydrogène,
- R¹, R², R³, R⁴ sont des groupements -OH.

La présente invention concerne en outre des dérivés répondant à la formule (I), dans laquelle :
- X¹, X², X³, X⁴, X⁵, X⁶, X⁷, X⁸ sont des atomes de chlore,
- R¹, R², R³, R⁴ sont soit des atomes de chlore, soit des groupements -OH.

La présente invention concerne également un dérivé de porphyrine, notamment bactériochlorine, de formule : 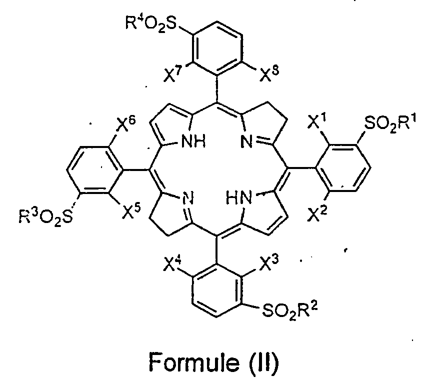 dans laquelle :
- X¹, X², X³, X⁴, X⁵, X⁶, X⁷, X⁸ sont chacun, identiques ou différents, choisis parmi les atomes d'halogène, et/ou les atomes d'hydrogène ;
- R¹, R², R³, R⁴ sont chacun, identiques ou différents, choisis parmi les groupements : -OH, acides aminés, -OR et -NHR et/ou atome de chlore, dans lesquels R est un groupement alkyle ayant de 1 à 12 atomes de carbone.

Un avantage de ce type de dérivés, et tel que pour la cas des chlorines décrites plus haut, réside dans le fait qu'ils sont aussi amphiphiles.

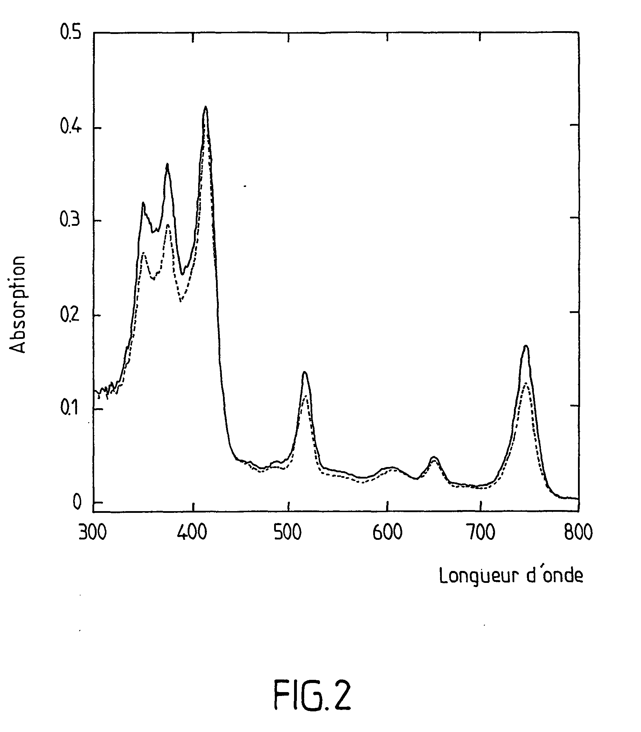

Un autre avantage de ces dérivés, notamment la bactériochlorine répondant à la formule (II), est le fait d'absorber la lumière dans la région du rouge à 750 nm, tel qu'illustré à la figure 2. A cette longueur d'onde, les effets d'écran provoqués par les tissus humains, notamment par l'hémoglobine, sont moindres, ce qui permet d'augmenter la « quantité » de lumière qui atteint les agents photosensibilisants à l'intérieur des cellules.

La présente invention concerne également les dérivés répondant à la formule (II), dans laquelle :
- X¹, X², X³, X⁴, X⁵, X⁶, X⁷, X⁸ sont chacun, identiques ou différents, choisis parmi les atomes d'halogène, et/ou les atomes d'hydrogène, tels qu'au moins un des radicaux X de chaque paire X¹/X², X³/X⁴, X⁵/X⁶, X⁷/X⁸ est un atome d'halogène.

Comme pour le cas de la chlorine décrite plus haut, la présence des atomes d'halogène dans les positions ortho du groupement phenyl, augmente le rendement quantique de formation de l'état triplet, sans pour autant diminuer significativement sa durée de vie, et par conséquent, augmente également le rendement de formation de l'oxygène singulet.

La présente invention concerne également certains dérivés, conformément à la formule (11), dans laquelle :
- X², X⁴, X⁶ et X⁸ sont soit des atomes de chlore, soit des atomes de fluor,
- X¹, X³, X⁵ et X⁷ sont des atomes d'hydrogène,
- R¹, R², R³, R⁴ sont des atomes de chlore,

Selon une autre variante de la présente invention, et conformément à la formule (II) :
- X², X⁴, X⁶ et X⁸ sont soit des atomes de chlore, soit des atomes de fluor,
- X¹, X³, X⁵ et X⁷ sont des atomes d'hydrogène,
- R¹, R², R³, R⁴ sont des groupements -OH.

La présente invention concerne en outre des dérivés répondant à la formule (II), dans laquelle :
- X¹, X², X³, X⁴, X⁵, X⁶, X⁷, X⁸ sont des atomes de chlore,
- R¹, R², R³, R⁴ sont soit des atomes de chlore, soit des groupements -OH.

La présence des atomes d'halogène dans les positions ortho du groupement phenyl, rend également le macrocycle tetrapyrrolique et ses dérivés plus stables.

Selon la présente invention, les atomes d'halogène sont le fluor et/ou le chlore et/ou le brome.

Un autre but de la présente invention consiste dans le procédé de préparation des dérivés tels que décrits ci-dessus comprenant les étapes suivantes :
- une étape de chlorosulfonation de la correspondante porphyrine halogénée,
- une étape de réduction de la porphyrine halogénée et chlorosulfonée à la chlorine et/ou à la bactériochlorine avec de l'hydrazide et dans la présence de bases encombrées organiques,
- une étape d'accouplement entre le groupement chlorosulfoné et des amines et/ou acides aminés et/ou alcools, ou l'hydrolyse du chlore du groupement chlorosulfoné avec de l'eau

A titre d'exemple, non limitatif, la synthèse chimique du dérivé répondant à la formule (I) dans laquelle :
- X², X⁴, X⁶ et X⁸ sont des atomes de chlore ;
- X¹, X³, X⁵ et X⁷ sont des atomes d'hydrogène,
- R¹, R², R³, R⁴ sont des groupements -OH,
a été réalisée comme suit :
5,10,15,20-tetrakis(2-chloro-3-chlorosulfonylphényl)porphyrine (0.0658 g, 6.55 x 10⁻⁵ mol) a été dissout dans de la N,N-dimethylformamide et/ou toluène, et/ou pyridine et/ou picoline (50 mL) séchée et distillée auparavant, et la température du mélange réactionnel a été portée de 50 °C à 150 °C. Ensuite, une solution de p-toluènesulfonyl hydrazide (5.5 x 10⁻⁵ mol) et une base encombrée organique, telle que le DABCO et/ou le DBU dans DMF (5mL) a été additionnée au mélange réactionnel. Le mélange a été laissé sous agitation à 150 °C dans une atmosphère d'azote et en absence de lumière. L'évolution de la réaction a été suivie par spectroscopie du visible jusqu'à ce que la bande Q à 650 nm atteigne son maximum.

Ensuite, le solvant a été évaporé et le mélange réactionnel a été traité et le produit obtenu a été hydrolysé avec de l'eau et purifié par extraction solvant-solvant. Après recristallisation avec un mélange méthanol/dichlométhane, le produit a été obtenu avec un rendement de 80 %.
Spectroscopie de masse (FAB), m/z : 1075 (pique central).
Vis-UV (solution tampon NaOH, KH₂PO₄), longueur d'onde maximum (en nm) : 414, 516, 542, 598, 649.
Rendement quantique de fluorescence : 0.04
Temps de vie de l'état triplet dans une solution aqueuse saturée d'azote : 235 µs
Temps de vie de l'état triplet dans une solution aqueuse saturée d'air : 3,88 µs
Rendement quantique de formation de l'oxygène singulet dans une solution aqueuse deuterée : 0.56.
Temps de vie de l'oxygène singulet : 65 µs dans de l'eau deuterée

De façon schématique la réaction peut être décrite comme suif :

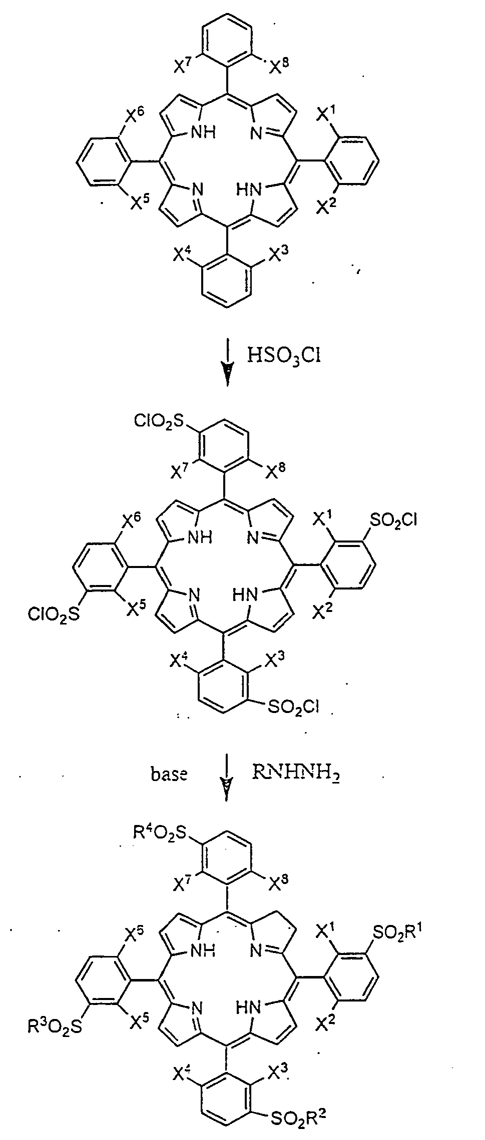

Egalement, à titre d'exemple, non limitatif, la synthèse chimique du dérivé répondant à la formule (II) dans laquelle :
- X², X⁴, X⁶ et X⁸ sont des atomes de chlore ;
- X¹, X³, X⁵ et X⁷ sont des atomes d'hydrogène,
- R¹, R², R³, R⁴ sont des groupements -OH,
   a été réalisée comme suit :
   5,10,15,20-tetrakis(2-chloro-3-chlorosulfonylphényl)porphyrine (0.0658 g, 6.55 x 10⁻⁵ mol) a été dissous dans de la N,N-diméthylformamide, et/ou pyridine et/ou picoline (50 mL) séchée et distillée auparavant, et la température a été portée de 50°C à 150°C. Ensuite, une solution de *p*-toluènesulfonyl hydrazide (130.0 x 10⁻⁵ mol) et DABCO dans du DMF (5mL) a été additionnée au mélange réactionnel. Le mélange a été laissé sous agitation à 150 °C dans une atmosphère d'azote et à l'abri de la lumière. L'évolution de la réaction a été suivie par spectroscopie du visible jusqu'à ce que la bande Q à 750 nm atteigne son maximum.

Ensuite, le solvant a été évaporé et le mélange réactionnel a été traité et le produit obtenu a été hydrolysé avec de l'eau pour donner le produit souhaité avec un rendement de 77 %.
Spectroscopie de masse (FAB), m/z : 1077 (pique central).
Vis-UV (solution tampon NaOH, KH₂PO₄), longueur d'onde maximum (en nm) : 353, 376, 486, 518, 604, 748.
Rendement quantique de fluorescence : 0.013
Temps de vie de l'état triplet dans une solution aqueuse saturée d'azote : 235 µs
Temps de vie de l'état triplet dans une solution aqueuse saturée d'air : 3,88 µs,
Rendement quantique de formation de l'oxygène singulet dans une solution aqueuse deuterée est supérieur à 30 %.
Temps de vie de l'oxygène singulet : 65 µs dans de l'eau deuterée.

Egalement, de façon schématique, la réaction peut être décrite comme suit :

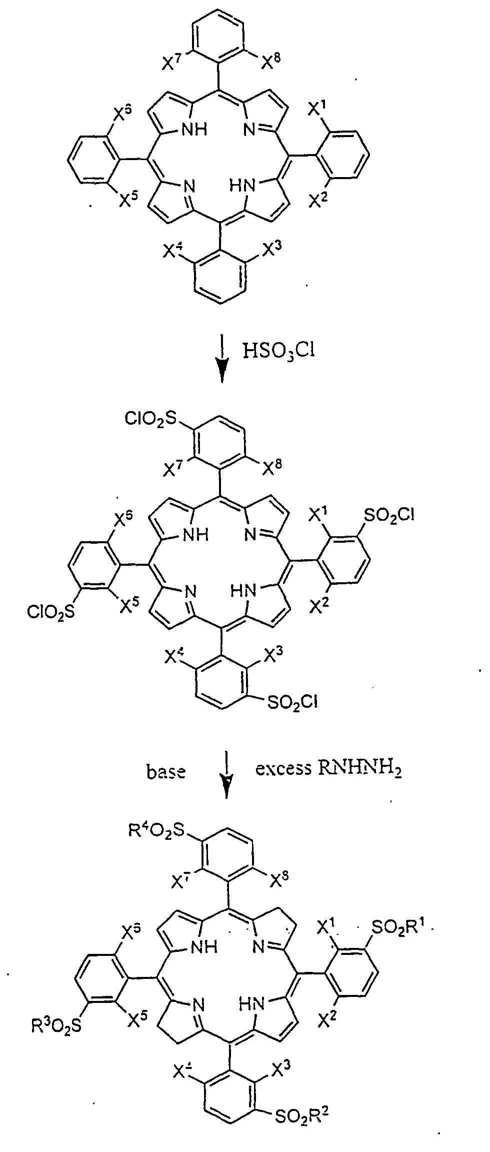

La 5,10,15,20-tetrakis(2-chloro-3-chlorosulfonylphenyl) porphyrine, utilisée comme porphyrine de départ dans la synthèse de la chlorine et de la bactériochlorine décrite ci-dessus, est connue de l'homme du métier et a été décrite dans la littérature, notamment dans le Journal of Heterocyclic Chemistry, 28 : 635 (1991*).*

Selon la présente invention, les rendements quantiques de formation de l'oxygène singulet ont été obtenus par comparaison entre l'intensité de phosphorescence de l'oxygène singulet à une longueur d'onde de 1270 nm dans une solution contenant l'agent photosensibilisant à étudier et l'intensité de phosphorescence obtenue à partir d'une autre solution présentant la même absorptivité à la même longueur d'onde, et contenant un agent photosensibilisant de référence.

Selon la présente invention, l'agent photosensibilisant de référence utilisé est la *meso*-tetrakis(3-sulfophenyl)porphyrine (TPPS) dont la valeur pour le rendement quantique de formation de l'oxygène singulet dans l'eau deuterée est de 0.64 à un pH de 7.4 (photochem. Photobiol, 70 : 391, 1999).

La présente invention concerne également un médicament anticancéreux et/ou antiviral et/ou antimicrobien à usage humain ou animal présentant comme principe actif principal un ou plusieurs composés tels que décrits ci-dessous.

Ce type de médicament, utilisé notamment en thérapie photodynamique, peut contenir aussi un ou plusieurs excipients pharmaceutiquement acceptables.

## Revendications

1. Dérivés de porphyrine, notamment chlorine, de formule 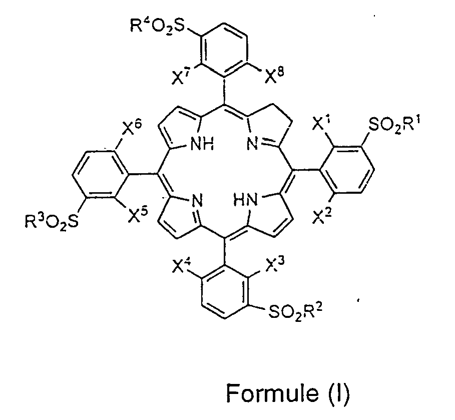 dans laquelle :
- X¹, X², X³, X⁴, X⁵, X⁶, X⁷, X⁸ sont chacun, identiques ou différents, choisis parmi les atomes d'halogène, et/ou des atomes d'hydrogène, tels qu'au moins un des radicaux X de chaque paire X¹/X², X³/X⁴, X⁵/X⁶, X⁷/X⁸ soit un atome d'halogène,
- R¹, R², R³, R⁴ sont chacun, identiques ou différents, choisis parmi les groupements : -OH, acides aminés, -OR et -NHR et/ou atome de chlore, dans lesquels R est un groupement alkyle ayant de 1 à 12 atomes de carbone.

2. Dérivés selon la revendication 1, répondant à la formule (I), dans laquelle :
- X², X⁴, X⁶ et X⁸ sont des atomes de chlore ;
- X¹, X³, X⁵ et X⁷ sont des atomes d'hydrogène ;
- R¹, R², R³, R⁴ sont des atomes de chlore.

3. Dérivés selon la revendication 1, répondant à la formule (I), dans laquelle :
- X², X⁴, X⁶ et X⁸ sont des atomes de fluor ;
- X¹, X³, X⁵ et X⁷ sont des atomes d'hydrogène ;
- R¹, R², R³, R⁴ sont des atomes de chlore.

4. Dérivés selon la revendication 1, répondant à la formule (1), dans laquelle :
- X², X⁴, X⁶ et X⁸ sont des atomes de chlore ;
- X¹, X³, X⁵ et X⁷ sont des atomes d'hydrogène ;
- R¹, R², R³, R⁴ sont des groupements -OH.

5. Dérivés selon la revendication 1, répondant à la formule(I), dans laquelle :
- X², X⁴, X⁶ et X⁸ sont des atomes de fluor ;
- X¹, X³, X⁵ et X⁷ sont des atomes d'hydrogène ;
- R¹, R², R³, R⁴ sont des groupement -OH.

6. Dérivés selon la revendication 1, répondant à la formule (I), dans laquelle :
- X¹, X², X³, X⁴, X⁵, X⁶, X⁷, X⁸ sont des atomes de chlore ;
- R¹, R², R³, R⁴ sont des atomes de chlore.

7. Dérivés selon la revendication 1,répondant à la formule (I) dans laquelle :
- X¹, X², X³, X⁴, X⁵, X⁶, X⁷, X⁸ sont des atomes de chlore ;
- R¹, R², R³, R⁴ sont des groupements -OH.

8. Dérivés de porphyrine, notamment bactériochlorine, de formule 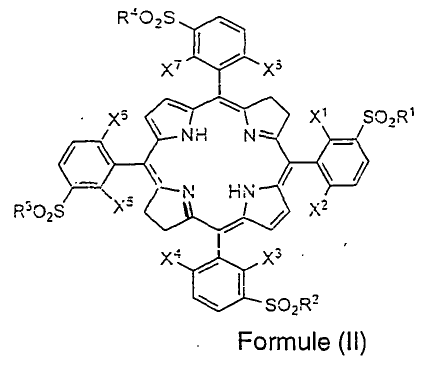 dans laquelle :
- X¹, X², X³, X⁴, X⁵, X⁶, X⁷, X⁸ sont chacun, identiques ou différents, choisis parmi les atomes d'halogène, et/ou des atomes d'hydrogène ;
- R¹, R², R³, R⁴ sont chacun, identiques ou différents, choisis parmi les groupements : -OH, acides aminés, -OR et -NHR et/ou atome de chlore, dans lesquels R est un groupement alkyle ayant de 1 à 12 atomes de carbone.

9. Dérivés selon la revendication 8, répondant à la formule (II), dans laquelle :
- X¹, X², X³, X⁴, X⁵, X⁶, X⁷, X⁸ sont chacun, identiques ou différents, choisis parmi les atomes d'halogène et/ou les atomes d'hydrogène, tels qu'au moins un des radicaux X de chaque paire X¹/X², X³/X⁴, X⁵/X⁶, X⁷/X⁸ soit un atome d'halogène.

10. Dérivés selon la revendication 8, répondant à la formule (II), dans laquelle :
- X², X⁴, X⁶ et X⁸ sont des atomes de chlore ;
- X¹, X³, X⁵ et X⁷ sont des atomes d'hydrogène ;
- R¹, R², R³, R⁴ sont des atomes de chlore.

11. Dérivés selon la revendication 8, répondant à la formule (II), dans laquelle,
- X², X⁴, X⁶ et X⁸ sont des atomes de fluor ;
- X¹, X³, X⁵ et X⁷ sont des atomes d'hydrogène ;
- R¹, R², R³, R⁴ sont des atomes de chlore.

12. Dérivés selon la revendication 8, répondant à la formule (II), dans laquelle :
- X², X⁴, X⁶ et X⁸ sont des atomes de chlore ;
- X¹, X³, X⁵ et X⁷ sont des atomes d'hydrogène ;
- R¹, R², R³, R⁴ sont des groupements -OH.

13. Dérivés selon la revendication 8, répondant à la formule (II), dans laquelle :
- X², X⁴, X⁶ et X⁸ sont des atomes de fluor ;
- X¹, X³, X⁵ et X⁷ sont des atomes d'hydrogène ;
- R¹, R², R³, R⁴ sont des groupements -OH.

14. Dérivés selon la revendication 8, répondant à la formule (II), dans laquelle :
- X¹, X², X³, X⁴, X⁵, X⁶, X⁷, X⁸ sont des atomes de chlore ;
- R¹, R², R³, R⁴ sont des atomes de chlore.

15. Dérivés selon la revendication 8, répondant à la formule (II), dans laquelle :
- X¹, X², X³, X⁴, X⁵, X⁶, X⁷, X⁸ sont des atomes de chlore ;
- R¹, R², R³, R⁴ sont des groupements -OH.

16. Dérivés de porphyrine selon l'une quelconque des revendications 1 ou 8, **caractérisés par le fait que** les atomes d'halogène sont le fluor et/ou le chlore et/ou le brome.

17. Procédé de préparation des dérivés selon l'une quelconques des revendications précédentes, **caractérisé par le fait qu'**il comprend :
- une étape de réduction de la correspondante porphyrine halogénée et chlorosulfonée à la chlorine et /ou à la bactériochlorine avec de l'hydrazide et des bases encombrées organiques ;
- une étape d'accouplement entre le groupement chlorosulfoné et des amines et/ou des acides aminés et/ou des alcools.

18. Procédé de préparation des dérivés selon l'une quelconques des revendications précédentes, **caractérisé par le fait qu'**il comprend :
- une étape de réduction de la correspondante porphyrine halogénée et chlorosulfonée à la chlorine et /ou à la bactériochlorine avec de l'hydrazide et des bases encombrées organiques ;
- une étape d'hydrolyse du chlore du groupement chlorosulfoné.

19. Médicament anticancéreux et/ou antiviral et/ou anti-microbien à usage humain ou animal présentant comme principe actif principal un ou plusieurs composés selon les revendications 1 à 16.

## Claims

1. Porphyrine derivatives, in particular chlorine, with the following formula: 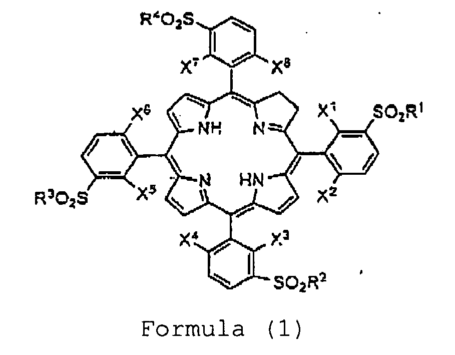 where :
- X¹, X², X³, X⁴, X⁵, X⁶, X⁷, X⁸ are each, identical or different, chosen from among the halogen atoms, and/or hydrogen atoms, so that at least one of the X radicals from each pair X¹/X², X³/X⁴, X⁵/X⁶, X⁷/X⁸ is a halogen atom,
- R¹, R², R³, R⁴ are each, identical or different, chosen from among the groups: -OH, amino acids, - OR and -NHR and/or chlorine atom, wherein R is an alkyl group with 1 to 12 carbon atoms.

2. Derivatives according to claim 1, complying with formula (1), wherein:
- X², X⁴, X⁶, and X⁸ are chlorine atoms;
- X¹, X³, X⁵, and X⁷ are hydrogen atoms;
- R¹, R², R³, R⁴ are chlorine atoms.

3. Derivatives according to claim 1, complying with formula (1), wherein:
- X², X⁴, X⁶, and X⁸ are fluorine atoms;
- X¹, X³, X⁵, and X⁷ are hydrogen atoms;
- R¹, R², R³, R⁴ are chlorine atoms.

4. Derivatives according to claim 1, complying with formula (1), wherein:
- X², X⁴, X⁶, and X⁸ are chlorine atoms;
- X¹, X³, X⁵ , and X⁷ are hydrogen atoms;
- R¹, R², R³, R⁴ are -OH groups.

5. Derivatives according to claim 1, complying with formula (1), wherein:
- X², X⁴, X⁶, and X⁸ are fluorine atoms;
- X¹, X³, X⁵, and X⁷ are hydrogen atoms;
- R¹, R², R³, R⁴ are -OH groups.

6. Derivatives according to claim 1, complying with formula (1), wherein:
- X¹, X², X³, X⁴, X⁵, X⁶, X⁷, X⁸ are chlorine atoms;
- R¹, R² R³, R⁴ are chlorine atoms.

7. Derivatives according to claim 1, complying with formula (1), wherein:
- X¹, X², X³, X⁴, X⁵, X⁶, X⁷, X⁸ are chlorine atoms;
- R¹, R², R³, R⁴ are -OH groups.

8. Porphyrine derivatives, in particular bacteriochlorine, with the following formula: 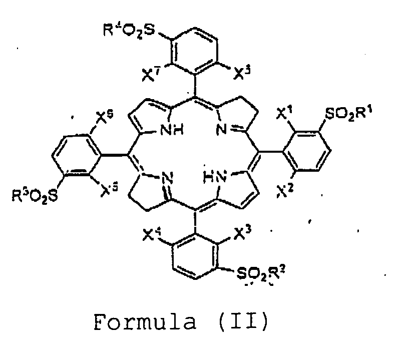 where:
- X¹, X², X³, X⁴, X⁵, X⁶, X⁷, X⁸ are each, identical or different, chosen from among the halogen atoms, and/or hydrogen atoms;
- R¹, R², R³, R⁴ are each, identical or different, chosen from among the groups: -OH, amino acids, - OR and -NHR and/or chlorine atom, wherein R is an alkyl group with 1 to 12 carbon atoms.

9. Derivatives according to claim 8, complying with formula (II), wherein:
- X¹, X², X³, X⁴, X⁵, X⁶, X⁷, X⁸ are each, identical or different, chosen from among the halogen atoms, and/or hydrogen atoms, so that at least one of the X radicals from each pair X¹/X², X³/X⁴, X⁵/X⁶, X⁷/X⁸ is a halogen atom,

10. Derivatives according to claim 8, complying with formula (II), wherein:
- X², X⁴, X⁶, and X⁸ are chlorine atoms;
- X¹, X³, X⁵, and X⁷ are hydrogen atoms;
- R¹, R², R³, R⁴ are chlorine atoms.

11. Derivatives according to claim 8, complying with formula (II), wherein:
- X², X⁴, X⁶, and X⁸ are fluorine atoms;
- X¹, X³, X⁵, and X⁷ are hydrogen atoms;
- R¹, R², R³, R⁴ are chlorine atoms.

12. Derivatives according to claim 8, complying with formula (II), wherein:
- X², X⁴, X⁶, and X⁸ are chlorine atoms;
- X¹, X³, X⁵, and X⁷ are hydrogen atoms;
- R¹, R², R³, R⁴ are -OH groups.

13. Derivatives according to claim 8, complying with formula (II), wherein:
- X², X⁴, X⁶, and X⁸ are fluorine atoms;
- X¹ X³, X⁵, and X⁷ are hydrogen atoms;
- R¹, R², R³, R⁴ are -OH groups.

14. Derivatives according to claim 8, complying with formula (II), wherein:
- X¹, X³, X⁵, and X⁷ are chlorine atoms;
- R¹, R², R³, R⁴ are chlorine atoms.

15. Derivatives according to claim 8, complying with formula (II) wherein:
- X¹, X², X³, X⁴, X⁵, X⁶, X⁷, X⁸ are chlorine atoms;
- R¹, R², R³, R⁴ are -OH groups.

16. Porphyrine derivatives according to any one of claims 1 or 8, **characterised in that** the halogen atoms are fluorine and/or chlorine and/or bromine.

17. A method for the preparation of derivatives according to any one of the preceding claims, **characterised in that** it includes:
- a step of reduction of the corresponding prophyrine halogenated and chlorosulphonated with chlorine and/or bacteriochlorine with hydrazide and overloaded organic bases;
- a step of coupling between the chlorosulphonated group and the amines and/or amino acids and/or alcohols.

18. A method for the preparation of derivatives according to any one of the preceding claims, **characterised in that** it comprises :
- a step of reduction of the corresponding porphyrine halogenated and chlorosulphated with chlorine and/or the bacteriochlorine with hydrazide and overloaded organic bases;
- a step of hydrolysis of the chlorine of the chlorosulphonated group.

19. An anti-neoplastic and/or anti-viral and/or anti-microbal drug for human or animal use with one or several compound(s) according to claims 1 to 16 as an active ingredient.

## Patentansprüche

1. Porphyrinderivate, insbesondere Chlorin, der Formel 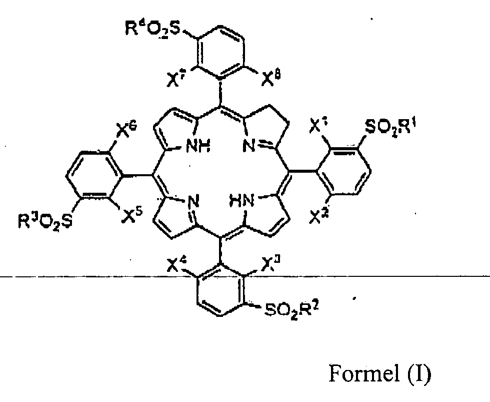 worin:
- X¹, X², X³, X⁴, X⁵, X⁶, X⁷, X⁸ jeweils identisch oder verschieden unter den Halogenatomen und/oder Wasserstoffatomen so ausgewählt sind, dass wenigstens eines der X-Radikale von jedem Paar X¹/X², X³/X⁴, X⁵/X⁶, X⁷/X⁸ ein Halogenatom sei,
- R¹, R², R³, R⁴ jeweils identisch oder verschieden unter den folgenden Gruppen ausgewählt sind: -OH, Aminosäuren, -OR und -NHR und/oder Chloratom, in denen R eine Alkylgruppe ist, die von 1 bis 12 Kohlenstoffatome hat.

2. Derivate nach Anspruch 1, die der Formel (I) entsprechen, worin:
- X², X⁴, X⁶ und X⁸ Chloratome sind;
- X¹, X³, X⁵ und X⁷ Wasserstoffatome sind;
- R¹, R², R³, R⁴ Chloratome sind.

3. Derivate nach Anspruch 1, die der Formel (I) entsprechen, worin:
- X², X⁴, X⁶ und X⁸ Fluoratome sind;
- X¹, X³, X⁵ und X⁷ Wasserstoffatome sind;
- R¹, R^{a}, R³, R⁴ Chloratome sind.

4. Derivate nach Anspruch 1, die der Formel (I) entsprechen, worin:
- X², X⁴, X⁶ und X⁸ Chloratome sind;
- X¹, X³, X⁵ und X⁷ Wasserstoffatome sind;
- R¹, R², R³, R⁴ -OH-Gruppen sind.

5. Derivate nach Anspruch 1, die der Formel (I) entsprechen, worin:
- X², X⁴, X⁶ und X⁸ Fluoratome sind;
- X¹, X³, X⁵ und X⁷ Wasserstoffatome sind;
- R¹, R², R³, R4 -OH-Gruppen sind.

6. Derivate nach Anspruch 1, die der Formel (I) entsprechen, worin:
- X¹, X², X³, X⁴, X⁵, X⁶, X⁷, X⁸ Chloratome sind;
- R¹, R², R³, R⁴ Chloratome sind.

7. Derivate nach Anspruch 1, die der Formel (I) entsprechen, worin;
- X¹, X², X³, X⁴, X⁵, X⁶, X⁷, X⁸ Chloratome sind;
- R¹, R², R³, R⁴ -OH-Gruppen sind.

8. Porphyrinderivate, insbesondere Bacteriochlorin, der Formel 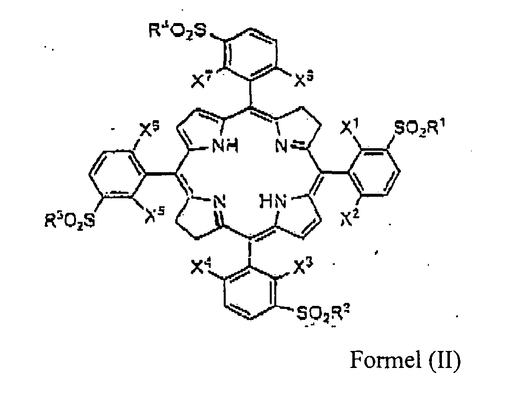 worin:
- X¹, X², X³, X⁴, X⁵, X⁶, X⁷, X⁸ jeweils identisch oder verschieden unter den Halogenatomen und/oder Wasserstoffatomen ausgewählt sind;
- R¹, R², R³, R⁴ jeweils identisch oder verschieden unter den folgenden Gruppen ausgewählt sind: -OH, Aminosäuren, -OR und -NHR und/oder Chloratom, in denen R eine Alkylgruppe ist, die von 1 bis 12 Kohlenstoffatome hat.

9. Derivate nach Anspruch 8, die der Formel (II) entsprechen, worin:
- X¹, X², X³, X⁴, X⁵, X⁶, X⁷, X⁸ jeweils identisch oder verschieden unter den Halogenatomen und/oder Wasserstoffatomen so ausgewählt sind, dass wenigstens eines der X-Radikale von jedem Paar X¹/X², X³/X⁴, X⁵/X⁶, X⁷/X⁸ ein Halogenatom sei.

10. Derivate nach Anspruch 8, die der Formel (II) entsprechen, worin:
- X², X⁴, X⁶ und X⁸ Chloratome sind;
- X¹, X³, X⁵ und X⁷ Wasserstoffatome sind;
- R¹, R², R³, R⁴ Chloratome sind.

11. Derivate nach Anspruch 8, die der Formel (II) entsprechen, worin:
- X², X⁴, X⁶ und X⁸ Fluoratome sind;
- X¹, X³, X⁵ und X⁷ Wasserstoffatome sind;
- R¹, R², R³, R⁴ Chloratome sind.

12. Derivate nach Anspruch 8, die der Formel (II) entsprechen, worin:
- X², X⁴, X⁶ und X⁸ Chloratome sind;
- X¹, X³, X⁵ und X⁷ Wasserstoffatome sind;
- R¹, R², R³, R⁴ -OH-Gruppen sind.

13. Derivate nach Anspruch 8, die der Formel (II) entsprechen, worin:
- X², X⁴, X⁶ und X⁸ Fluoratome sind;
- X¹, X³, X⁵ und X⁷ Wasserstoffatome sind;
- R¹ , R², R³, R⁴ -OH-Gruppen sind.

14. Derivate nach Anspruch 8, die der Formel (II) entsprechen, worin:
- X¹, X², X³, X⁴, X⁵, X⁶, X⁷, X⁸ Chloratome sind;
- R¹, R², R³, R⁴ Chloratome sind.

15. Derivate nach Anspruch 8, die der Formel (II) entsprechen, worin:
- X¹, X², X³, X⁴, X⁵, X⁶, X⁷, X⁸ Chloratome sind;
- R¹ R², R³, R⁴ -OH-Gruppen sind.

16. Porphyrinderivate nach irgendeinem der Ansprüche 1 oder 8, **dadurch gekennzeichnet, dass** die Halogenatome Fluor und/oder Chlor und/oder Brom sind.

17. Verfahren zur Herstellung der Derivate nach irgendeinem der vorgehenden Ansprüche, **dadurch gekennzeichnet, dass** es folgendes umfasst:
- einen Schritt zur Reduktion des entsprechenden halogenierten und chlorsulfonierten Porphyrins zu Chlorin und/oder zu Bacteriochlorin mit Hydrazid und gesättigten organischen Laugen;
- einen Schritt zur Verbindung zwischen der Chlorsulfongruppe und Aminen und/oder Aminosäuren und/oder Alkoholen.

18. Verfahren zur Herstellung der Derivate nach irgendeinem der vorgehenden Ansprüche, **dadurch gekennzeichnet, dass** es folgendes umfasst:
- einen Schritt zur Reduktion des entsprechenden halogenierten oder chlorsulfonierten Porphyrins zu Chlorin und/oder zu Bacteriochlorin mit Hydrazid und gesättigten organischen Laugen;
- einen Schritt zur Hydrolyse des Chlors der chlorsulfonierten Gruppe.

19. Krebsbekämpfendes und/oder antivirales und/oder antibakterielles Arzneimittel für Menschen und Tieren, das als Hauptwirkstoff eine oder mehrere Verbindungen nach einem der Ansprüche 1 bis 16 aufweist.
